# EUROPÄISCHE PATENTSCHRIFT

(11) **EP 1 142 888 B1**
(45) Veröffentlichungstag und Bekanntmachung des Hinweises auf die Patenterteilung: **08.09.2004**
(21) Anmeldenummer: 01106444.1
(22) Anmeldetag: 23.03.2001
(51) Int. Cl.: C07D 333/32

(54) **Verfahren zur Herstellung von Dialkoxythiophenen und Alkylendioxythiophenen**
Process for the preparation of Dialcoxythiophenes and Alkylendioxythiophenes
Procédé pour la préparation de dialcoxy thiophène et alcoylèndioxy thiophène

(30) Priorität: 04.04.2000 DE 10016723
(43) Veröffentlichungstag der Anmeldung: 10.10.2001
(73) Patentinhaber: BAYER AG, 51368 Leverkusen (DE)
(72) Erfinder: Rauchschwalbe, Günter, Dr., 51375 Leverkusen (DE); Jonas, Friedrich, Dr., 52066 Aachen (DE)

(56) Entgegenhaltungen:
- WO-A-95/24373
- US-A- 2 453 103
- MERZ,A. ET AL.: "IMPROVED PREPARATION OF 3,4-DIMETHOXYTHIOPHENE" J.PRAKT.CHEM, Bd. 338, 1996, Seiten 672-674, XP001000173 LEIPZIG
- HEYWANG G ET AL: "POLY(ALKYLENEDIOXYTHIOPHENE)S-NEW, VERY STABLE CONDUCTING POLYMERS**" ADVANCED MATERIALS,DE,VCH VERLAGSGESELLSCHAFT, WEINHEIM, Bd. 4, Nr. 2, 1. Februar 1992 (1992-02-01), Seiten 116-118, XP000306687 ISSN: 0935-9648
- OVERBERGER,C.G. ET AL.: "The Prparation of 3,4-Dimethoxy-2,5-dicarbethoxythiphene. 3,4-Dimethoxythiphene." J.AM.CHEM.SOC., Bd. 73, 1951, Seiten 2956-2957, XP001005623 WASHINGTON
- COFFEY, M. ET AL.: "A facile sytnesis of 3,4-dialkyoxthiphenes " SYNTHETIC COMMUN., Bd. 26, Nr. 11, 1996, Seiten 2205-2212, XP001005513 NEW YORK

## Beschreibung

Die Erfindung betrifft ein Verfahren zur Herstellung von Dialkoxythiophenen und Alkylendioxythiophenen durch Decarboxylierung von Dialkoxythiophen-dicarbonsäuren bzw. Alkylendioxythiophen-dicarbonsäuren in Lösungsmitteln, die einen höheren Siedepunkt als das Produkt besitzen und die keine Stickstoff-Basen enthalten.

WO-A 95/24373 beschreibt ein Verfahren zur Monodecarboxylierung von halogenierten aromatischen Dicarbonsäuren in polar aprotischen Lösungsmitteln wie beispielsweise Sulfolan, N,N-Dimethylacetamid oder N-Methylpyrolidon bei Temperaturen von 40 bis 80°C, wobei das Produkt durch Ausfällung isoliert wird, jedoch ist weder eine Didecarboxylierung unter den dort beschriebenen Bedingungen mit ausreichender Ausbeute zu erzielen noch ist das Verfahren für die Decarboxylierung von Dialkoxy- bzw. Alkylendioxythiophen-dicarbonsäuren geeignet.

US-A-2 453 103 (DuPont, 1948) beschreibt die thermische Decarboxylierung von 3,4-Dimethoxythiophen-2,5-dicarbonsäure in Chinolin bei 180-185°C unter Zusatz von speziellem Cu-Pulver.

Die Anwesenheit von Aminen im Endprodukt, auch in Spuren, muss jedoch vermieden werden, da sie im folgenden Schritt stören.

Daher erfolgt die Aufarbeitung durch Auswaschen mit Wasser und Säure: Das basische Chinolin gelangt dabei als Salz ins Abwasser, wodurch die Umwelt belastet wird oder aber ein zusätzlicher, aufwendiger Schritt erforderlich wird, das basische Chinolin wieder aus der wässrigen Phase zurückzugewinnen. Auch der Ersatz des Cu-Katalysators durch Cu-Cr-Oxid (siehe E. Fager, J. Amer. Chem. Soc. 67 (1945), 2217-8) führt nicht zu besseren Ergebnissen (58 % Ausbeute nach Decarboxylieren in Chinolin bei 180°C und wässrig-saurer Aufarbeitung).

Das gilt auch für die Vorschrift nach M. Coffey et al., Synthetic Communications 26 (11), 2205-12 (1996), Methode 2, aus der obendrein zu entnehmen ist, dass Cu (als Kupferbronze) vorteilhaft in erheblichen Mengen verwendet werden muss, nämlich in 1 Teil auf 4 Teile Dicarbonsäure. Die erforderliche Temperatur von 180 - 200°C erfordert erheblichen Energieaufwand und Apparaturen, die nicht überall zur Ver fügung stehen. Die Ausbeute erreicht für 3,4-Ethylendioxythiophen (EDT) nur 54 %, das ist für eine industrielle Anwendung ungenügend.

Das gilt in vermehrtem Maße auch für die Methode aus US-A-2 453 103. Sie erfordert 2- bis 4-stündiges Erhitzen bis zum Schmelzpunkt der eingesetzten Dicarbonsäure. Die EDT-Dicarbonsäure schmilzt erst bei Temperaturen oberhalb von 300°C; dabei tritt erhebliche Bildung teeriger Massen auf, wodurch die in Methode 1 beschriebene Reinigung durch Umkristallisation sehr schwierig und verlustreich ist. In der Tat wird diese Methode in der o.g. Publikation auch nicht für 3,4-EDT-dicarbonsäure beschrieben.

3,4-Dimethoxythiophen wurde auch schon durch Decarboxylierung von 3,4-Dimethoxy-thiophen-2,5-dicarbonsäure (in Anwesenheit von Cu-Pulver, bei 180-190°C) ohne Lösungsmittel erhalten (C. Overberger, J. Am. Chem. Soc. 73 (1951), 2956-57). Die hohe Temperatur, die zur Reaktionsführung erforderlich ist, steht auch hier einer technischen Anwendung im Wege; für die Übertragung auf EDT gilt das im vorigen Abschnitt Gesagte.

In Abwesenheit von Verdünnungsmittel sowie von Metallkatalysator wird bei der rein thermischen Decarboxylierung von 3,4-Dimethoxythiophen-dicarbonsäure zu Dimethoxythiophen bei 250°C nur eine Ausbeute von 65 % erreicht (A. Merz, Chr. Rehm, J. prakt. Chem. 338 (1996), 672-4; es wird dabei ein Produktgemisch erhalten, das noch nachträglich aufwendig, d.h. in mehreren Schritten, aufgetrennt werden muss).

EDT und ähnliche 3,4-Dialkoxythiophene sind sehr wertvolle Materialien zur Herstellung leitfähiger Polymere (siehe z.B. G. Heywang, F. Jonas, Adv. Mater. 1992, 4, 116; F. Jonas, L. Schrade, Synthetic Metals, 41-43 (1991), 831-836).

Ein direkter Weg zu solchen Thiophenen führt über die Kondensation von Thiodiessigsäureestern mit z.B. Oxalsäureestern über 3,4-Dihydroxythiophen-dicarbonsäure-ester, der alkyliert, verseift und decarboxyliert werden kann (siehe Hinsberg, Chem. Ber. 43, (1910), 904; sowie G. Heywang, F. Jonas, Advanced Materials 4 (1992), 116).

Es wurde nun eine besondere Ausführungsform dieser Decarboxylierungsreaktion gefunden.

Gegenstand der Erfindung ist daher ein Verfahren zur Herstellung von 3,4-Dialkoxy- oder 3,4-Alkylendioxy-thiophenen der allgemeinen Formeln I oder II 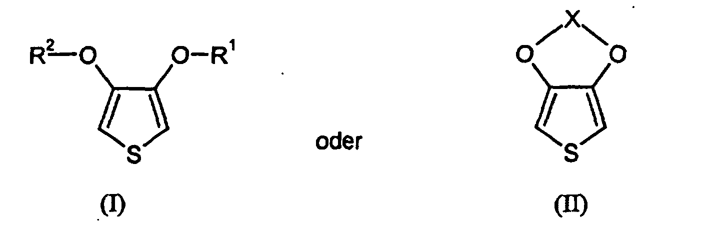 durch Decarboxylierung der zugrundeliegenden 3,4-Dialkoxy- bzw. 3,4-Alkylendioxy-2,5-thiophen-dicarbonsäuren der allgemeinen Formeln III oder IV 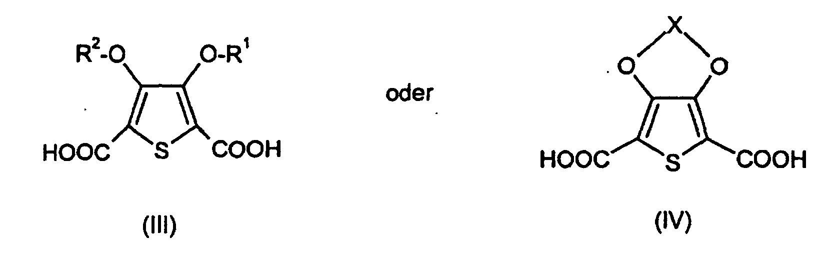 wobei
R¹ und R² - geradkettiges oder verzweigtes - Alkyl mit 1 bis 15 C-Atomen bedeuten
und
wobei
X für gegebenenfalls substituiertes -(CH₂)ₙ- steht und n eine ganze Zahl zwischen 1 und 12 bedeutet,
dadurch gekennzeichnet,
- dass die Decarboxylierung in einem Lösungs- bzw. Verdünnungsmittel durchgeführt wird, das einen höheren Siedepunkt aufweist als das decarboxylierte Produkt und das nicht zur Klasse der aromatischen Amine gehört, und
- dass das Endprodukt durch Destillation aus diesem höher siedenden Lösungs- bzw. Verdünnungsmittel abgetrennt wird.

Besonders geeignet ist das erfindungsgemäße Verfahren zur Herstellung von 3,4-Ethylendioxythiophen (EDT, IUPAC-Name: 2,3-Dihydro-thieno[3,4-b] [1,4]dioxine): 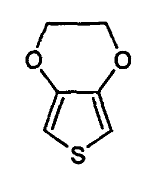 sowie von daraus abgeleiteten alkylsubstituierten Verbindungen wie z.B.: 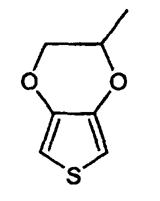 oder von 3,4-Dimethoxythiophen.

Das neue Verfahren gestattet die Durchführung der gewünschten Decarboxylierung von Dialkoxythiophen-dicarbonsäuren zu Dialkoxythiophenen sowie die Aufarbeitung der erhaltenen Produkte in einer eleganten und einfachen Verfahrensweise. Die gewünschten Produkte werden dabei in sehr guter Ausbeute erhalten.

Die Erfindung ist dadurch gekennzeichnet, dass das Edukt, die Dialkoxythiophendicarbon-säure in einem polaren Lösungs- bzw. Verdünnungsmittel suspendiert wird, das einen höheren Siedepunkt aufweist als das gewünschte Dialkoxythiophen. Vorzugsweise hat das Lösungs- bzw. Verdünnungsmittel einen um wenigstens 5°C höheren Siedepunkt. Wird das Edukt in wässriger Lösung oder Suspension eingesetzt, so kann das Wasser in einem ersten Schritt durch Erhitzen und Abdestillieren in situ abdestilliert werden. Ein gesonderter Trocknungsschritt, z.B. in einem Trockenschrank oder einem Schaufeltrockner, kann dadurch entfallen.

Im Anschluss daran wird die Decarboxylierung bei erhöhter Temperatur durchgeführt und schließlich das Produkt während oder nach der Decarboxylierung aus dem Lösungs- bzw. Verdünnungsmittel abdestilliert. Die Bedingungen der Destillation richten sich nach den physikalischen Eigenschaften von Produkt und Verdünnungsmittel sowie nach den Reinheitsanforderungen. So kann man z.B. zunächst ein Verdünnungsmittel enthaltendes Produktgemisch direkt aus dem Reaktionskessel abdestillieren und danach einer Rektifikation unterwerfen; man kann aber auch in einer geeigneten Apparatur das Produkt, gegebenenfalls über eine Kolonne, abdestillieren.

In der Regel genügt eine einzelne Destillation über eine Trennkolonne, um besonders reine Produkte zu erhalten.

Dabei dient das Verdünnungsmittel unter anderem dazu, die über die Kesselwand zugeführte Wärme abzuführen und zu verteilen, so dass lokale Überhitzungen vermieden werden.

Die Decarboxylierungsreaktion kann ohne Katalysator, d.h. bei einer Temperatur von 170 - 260°C durchgeführt werden; in einer bevorzugten Ausführungsform wird sie in Anwesenheit eines Katalysators durchgeführt, wobei deutlich niedrigere Temperaturen genügen, z.B. in einem Bereich von 100 - 180°C; bevorzugt sind Temperaturen zwischen 120 und 170°C, besonders bevorzugt zwischen 130 und 160°C; als Katalysator können Schwermetallverbindungen dienen, z. B. Kupfersalze.

Als erfindungsgemäße Lösungs- bzw. Verdünnungsmittel können beispielsweise Silikonöle, Ketone, Ester, Ether, Sulfoxide, Sulfone oder Alkohole verwendet werden. Stickstoffbasen wie Chinolin sind jedoch ungeeignet, da sie auch in Spuren die Qualität der Endprodukte beeinträchtigen.

Besonders geeignet sind z.B. Baysilone® (Handelsprodukte der Bayer AG), Polyethylenglykole, Phthalsäureester, Diarylether, Tetramethylensulfon, Diarylsulfone und Diarylsulfoxide.

Ganz besonders geeignet sind Baysilone® sowie Polyethylenglykol 300 und 400, Phthalsäure-dibutylester, Ditolylether, Diphenylsulfon, Diphenylsulfoxid und Tetramethylensulfon.

Bei geeigneter Durchführung kann - in Abhängigkeit von der Reinheit des eingesetzten Eduktes - frisches Edukt in den Kesselrückstand nachgesetzt werden, der den größten Teil des Verdünnungsmittels enthält, und ein neuer Reaktionszyklus durchgeführt werden. Nach mehreren Zyklen wird dann das Verdünnungsmittel z.B. durch Destillation, durch Zusatz von Wasser oder auf andere Weise von dunklen Nebenprodukten abgetrennt und kann in erheblichem Maße wiedergewonnen und erneut eingesetzt werden, was die Wirtschaftlichkeit des Verfahrens erheblich verbessert.

Die Anwesenheit eines Verdünnungsmittel erleichtert obendrein noch die Abtrennung von Nebenkomponenten, was die Reinigung des Kessels nach der Produktionskampagne erleichtert.

Als katalytisch wirksame Schwermetallverbindung, die die Decarboxylierung bei niedrigerer Temperatur gestattet, dient z.B. basisches Kupfercarbonat, Kupfersulfat, Kupferoxid oder Kupferhydroxyd.

In einer besonders geeigneten Ausführungsform wird feuchte Dialkoxythiophendicarbonsäure bzw. Alkylendioxythiophen-dicarbonsäure in das Verdünnungsmittel eingebracht, durch Erhitzen über den Siedepunkt von Wasser erhitzt und durch Abdestillieren von Wasser getrocknet; dann wird gegebenenfalls Schwermetallkatalysator zugegeben, durch Erhitzen auf die erforderliche Temperatur die Decarboxylierung durchgeführt, und dann (gegebenenfalls im Vakuum) das gewünschte Produkt abdestilliert. Diese Destillation kann zunächst ohne Trennleistung erfolgen, es kann aber auch bereits über eine Kolonne destilliert werden, so dass das Dialkoxythiophen bzw. Alkylendioxythiophen in der gewünschten Reinheit erhalten wird. Aus besonders hoch siedenden Verdünnungsmitteln kann auf dieser Stufe sogar ohne Trennstufe destilliert werden.

Gegebenenfalls anzuwendende weitere Reinigungsmethoden für rohe Destillate sind dem Fachmann bekannt. Besonders genannt seien das Waschen oder die Chromatographie.

Die gewählte Verfahrensweise hängt von äußeren Faktoren ab wie z.B. dem Siedeverhalten des Verdünnungsmittels im Vergleich zum Produkt, der zur Verfügung stehenden Apparatur bzw. von den erwünschten Taktzeiten.

Durch die geschilderten Varianten soll die Erfindung beschrieben, aber in keiner Weise limitiert werden.

### Beispiel 1:

### Unkatalysierte rein thermische Decarboxylierung

In einem Rührkolben legt man 450 g Dibutylphthalat vor und gibt 240 g 3,4-Ethylendioxythiophen-dicarbonsäure zu. Man legt Vakuum an (ca. 30 mbar) und erwärmt zunächst während 1 Stunde auf 150°C; dabei destilliert Wasser ab.

Man belüftet die Apparatur mit Stickstoff und erhitzt während 24 Stunden auf 240°C, bis die CO₂-Entwicklung beendet ist. Man kühlt wieder ab, legt Vakuum an und destilliert 3,4-Ethylendioxythiophen bei 0,1 mbar ab.

Man erhält 118 g Produkt (ca. 80 % d.Th.).

### Beispiel 2:

### Katalysierte Decarboxylierung

In einem Rührkolben legt man 1200 ml Tetramethylensulfon ("Sulfolan") vor und gibt 690 g EDT-dicarbonsäure (wasserfeucht; die Gehaltsbestimmung erfolgte durch Flüssigkeitschromatographie) und 66 g basisches Kupfercarbonat zu. Man erhitzt bei einem Innendruck von ca. 20 mbar auf eine Innentemperatur von 80°C und destilliert das Wasser ab. Man belüftet mit Stickstoff und erhöht die Temperatur auf 140°C. Man rührt bei dieser Temperatur während 8 Stunden, bis die Gasentwicklung beendet ist. Dann kühlt man etwas ab, legt wieder Vakuum an (ca. 20 mbar) und destilliert bei einer Innentemperatur von ca. 150°C 708 g EDT-Sulfolan-Gemisch ab.

In den Destillationsrückstand gibt man wieder die gleiche Menge feuchte EDT-Dicarbonsäure und etwas basisches Kupfercarbonat und ersetzt die abdestillierte Menge an Sulfolan durch frisches Sulfolan und verfährt wie beschrieben.

Dieses Nachsetzen erfolgte insgesamt dreimal.

Insgesamt erhielt man 3184 g EDT-Sulfolan-Gemisch, das lt. gaschromatographischer Analyse 1632 g reines EDT enthielt (95,6 % Ausbeute).

Durch Feindestillation über eine kurze Kolonne konnte daraus nach einem kurzen Vorlauf EDT in einer Ausbeute von 95 % (bezogen auf Einsatz), ein Nachlauf von mit Sulfolan kontaminiertem EDT (3 % bezogen auf Einsatz; wiederverwendbar im nächsten Ansatz) sowie 1550 g reines Sulfolan erhalten werden, das ebenfalls wiederverwendbar war.

Die Identität des Produktes wurde durch gaschromatographische Analyse bestätigt (Vergleich mit authentischer Ware).

Der Rückstand aus der Rohdestillation war ohne weiteres pumpbar und kann zur Entsorgung (z.B. durch Verbrennung) gegeben werden, es kann aber auch ein wesentlicher Teil des enthaltenen Sulfolans wiedergewonnen werden.

Die Zahl der bis zur Entsorgung möglichen Zyklen hängt i.w. von der Reinheit des eingesetzten Eduktes ab, da Verunreinigungen sich im Destillationssumpf anreichern und deren Menge das Verhalten des Sumpfes bestimmt.

### Beispiel 3:

### (vereinfachte Aufarbeitung)

Man verfuhr wie in Beispiel 2; nach erfolgter Decarboxylierung wurde EDT jedoch direkt über eine Kolonne abdestilliert.

Man erhielt sehr reines EDT in einer Ausbeute von 95 % d.Th.

### Beispiel 4:

### (Übertragung auf 3,4-Dimethoxythiophen)

In einem Rührkolben wurden 175 ml Sulfolan und 105 g wasserfeuchte, 70 %ige 3,4-Dimethoxythiophen-dicarbonsäure (73,5 g (trocken) / 0,316 mol) vorgelegt. Nach Zugabe von 10 g basischen Kupfercarbonates wurde innerhalb von 1 Stunde bei 85°C und 50 mbar trockendestilliert. Die Apparatur wurde mit Stickstoff belüftet. Bei einer Innentemperatur von 140°C wurde gerührt, bis die CO₂-Entwicklung beendet war (9 Stunden). Nach Zugabe von weiteren 3 g Kupfercarbonat wurde noch 3 Stunden nachgerührt. Dann wurde 3,4-Dimethoxythiophen bei 145°C Sumpf- und 130°C Kopftemperatur über eine kleine Kolonne abdestilliert.

Man erhielt 39,5 g Produkt (0,274 mol / 86,7 % d.Th.).

Die Identität wurde durch Massenspektrometrie sowie durch ¹H-NMR bestätigt.

### Beispiel 5:

### (Verwendung von Polyethylenglykol 300 als Lösungsmittel)

1,5 mol EDT-Dicarbonsäure wurde in Anwesenheit von 33 g basischem Kupfercarbonat in 600 ml Polyethylenglykol (MG 300) (statt in Sulfolan) innerhalb von 20 Stunden decarboxyliert; EDT wurde daraus über eine Destillationsbrücke abdestilliert und in einer Reinheit von 97,7 % und einer Ausbeute von 96 % d.Th. erhalten. Anhaftende Verunreinigungen (Glykol, Diglykol) wurden durch Waschen mit wenig Wasser daraus entfernt. Man erhielt EDT in einer Reinheit von >99%.

## Patentansprüche

1. Verfahren zur Herstellung von 3,4-Dialkoxy- oder 3,4-Alkylendioxy-thiophenen der allgemeinen Formeln I oder II 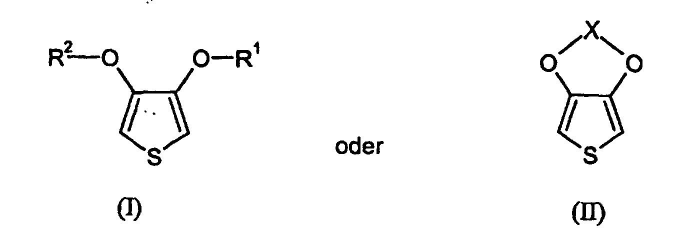 durch Decarboxylierung der zugrundeliegenden 3,4-Dialkoxy- bzw. 3,4-Alkylen-dioxy-2,5-thiophen-dicarbonsäuren der allgemeinen Formeln III oder IV 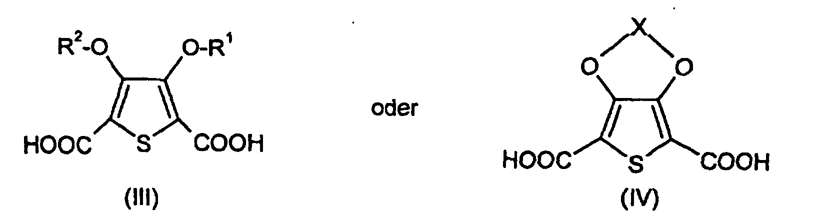 wobei
R¹ und R² - geradkettiges oder verzweigtes - Alkyl mit 1 bis 15 C-Atomen bedeuten
und
wobei
X für gegebenenfalls substituiertes -(CH₂)ₙ- steht und n eine ganze Zahl zwischen 1 und 12 bedeutet,
**dadurch gekennzeichnet,**
- **dass** die Decarboxylierung in einem Lösungs- bzw. Verdünnungsmittel durchgeführt wird, das einen höheren Siedepunkt aufweist als das decarboxylierte Produkt und das nicht zur Klasse der aromatischen Amine gehört, und
- **dass** das Endprodukt durch Destillation aus diesem höher siedenden Lösungs- bzw. Verdünnungsmittel abgetrennt wird.

2. Verfahren nach Anspruch 1, **dadurch gekennzeichnet, dass** das Lösungs- bzw. Verdünnungsmittel einen um wenigstens 5°C höheren Siedepunkt hat.

3. Verfahren nach Anspruch 1, **dadurch gekennzeichnet, dass** als Lösungs- bzw. Verdünnungsmittel Silikonöle, Ketone, Ester, Ether, Sulfoxide, Sulfone oder Alkohole verwendet werden.

4. Verfahren nach Anspruch 1, **dadurch gekennzeichnet, dass** die Decarboxylierung in Anwesenheit eines Schwermetallsalzes durchgeführt wird.

5. Verfahren nach Anspruch 4, **dadurch gekennzeichnet, dass** das Schwermetallsalz ein Kupfersalz ist.

6. Verfahren nach Anspruch 4 und 5, **dadurch gekennzeichnet, dass** die Decarboxylierung in einem Temperaturbereich zwischen 100 und 180°C durchgeführt wird.

7. Verfahren nach Anspruch 1, **dadurch gekennzeichnet, dass** als Produkte 3,4-Dimethoxythiophen oder 3,4-Ethylendioxythiophen hergestellt werden.

8. Verwendung der mit dem Verfahren nach Anspruch 1 erhaltenen Dialkoxy- bzw. Alkylendioxythiophene zur Herstellung leitfähiger Polymere.

## Claims

1. Process for the preparation of 3,4-dialkoxy- or 3,4-alkylenedioxythiophenes of the general formula I or II 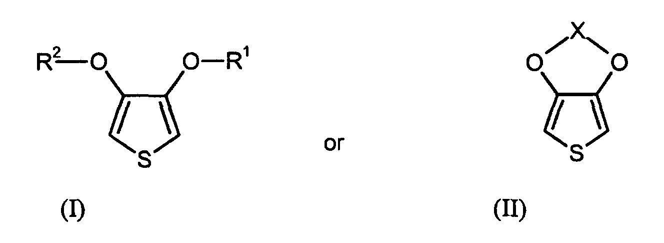 by decarboxylation of the parent 3,4-dialkoxy- or 3,4-alkylenedioxy-2,5-thiophenedicarboxylic acids respectively of the general formula III or IV 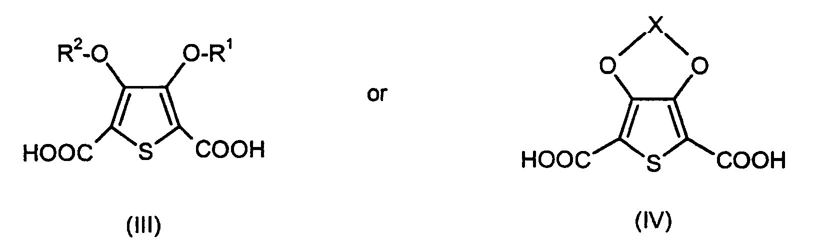 where
R¹ and R² - straight-chain or branched - are alkyl having 1 to 15 carbon atoms
and
where
X is optionally substituted -(CH₂)ₙ-, and n is an integer from 1 to 12,
**characterized in that**
- the decarboxylation is carried out in a solvent or diluent which has a higher boiling point than the decarboxylated product and does not belong to the class of the aromatic amines, and
- the end product is separated from this higher-boiling solvent or diluent by distillation.

2. Process according to Claim 1, **characterized in that** the solvent or diluent has a boiling point which is at least 5°C higher.

3. Process according to Claim 1, **characterized in that** the solvents or diluents used are silicone oils, ketones, esters, ethers, sulphoxides, sulphones or alcohols.

4. Process according to Claim 1, **characterized in that** the decarboxylation is carried out in the presence of a heavy-metal salt.

5. Process according to Claim 4, **characterized in that** the heavy-metal salt is a copper salt.

6. Process according to Claim 4 or 5, **characterized in that** the decarboxylation is carried out at a temperature in the range from 100 to 180°C.

7. Process according to Claim 1, **characterized in that** the product prepared is 3,4-dimethoxythiophene or 3,4-ethylenedioxythiophene.

8. Use of a dialkoxy- or alkylenedioxythiophene obtained using the process according to Claim 1 for the preparation of conductive polymers.

## Revendications

1. Procédé pour la préparation de 3,4-dialcoxy- ou 3,4-alkylènedioxy-thiophènes de formules générales (I) ou (II) 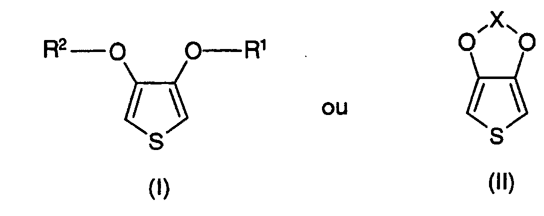 par décarboxylation des acides 3,4-dialcoxy- ou 3,4-alkylènedioxy-2,5-thiophène-dicarboxyliques correspondants de formules générales (III) ou (IV) 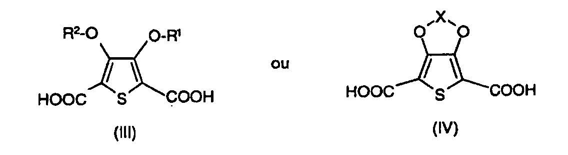 dans lesquelles
R¹ et R² représentent des groupes alkyle à chaîne droite ou ramifiée en C₁-C₁₅ et
X représente un groupe -(CH₂)ₙ- éventuellement substitué pour lequel n est un nombre entier allant de 1 à 12,
**caractérisé en ce que**
- la décarboxylation est réalisée dans un solvant ou diluant ayant un point d'ébullition supérieur à celui du produit décarboxylé et qui n'appartient pas à la classe des amines aromatiques, et
- le produit final est séparé du solvant ou diluant à point d'ébullition plus élevé par distillation.

2. Procédé selon la revendication 1, **caractérisé en ce que** le solvant ou diluant a un point d'ébullition supérieur d'au moins 5°C.

3. Procédé selon la revendication 1, **caractérisé en ce que** l'on utilise en tant que solvants ou diluants des huiles de silicone, des cétones, des esters, des éthers, des sulfoxydes, des sulfones ou des alcools.

4. Procédé selon la revendication 1, **caractérisé en ce que** la décarboxylation est réalisée en présence d'un sel de métal lourd.

5. Procédé selon la revendication 4, **caractérisé en ce que** le sel de métal lourd est un sel de cuivre.

6. Procédé selon les revendications 4 et 5, **caractérisé en ce que** la décarboxylation est réalisée dans l'intervalle de température de 100 à 180°C.

7. Procédé selon la revendication 1, **caractérisé en ce que** l'on prépare le 3,4-diméthoxythiophène ou le 3,4-éthylènedioxythiophène.

8. Utilisation des dialcoxy- ou alkylènedioxy-thiophènes obtenus par le procédé selon la revendication 1 pour la préparation de polymères conducteurs.
